# NEW EUROPEAN PATENT SPECIFICATION

(11) **EP 2 461 904 B2**
(45) Date of publication and mention of the opposition decision: **07.10.2026**
(45) Mention of the grant of the patent: 04.10.2017
(21) Application number: 10806914.7
(22) Date of filing: 28.07.2010
(51) Int. Cl.: B01J 23/38, B01J 23/70, B01J 37/02, C07C 17/25, C07C 19/08

(54) **HYDROGENATION CATALYST**
HYDRIERUNGSKATALYSATOR
CATALYSEUR D'HYDROGÉNATION

(30) Priority: 03.08.2009 US 534323
(43) Date of publication of application: 13.06.2012
(60) Divisional application: 17194512.4
(73) Proprietor: Honeywell International Inc., Morris Plains, NJ 07950 (US)
(72) Inventor: WANG, Haiyou, Amherst New York 14228 (US); TUNG, Hsueh Sung, Getzville New York 14068 (US); MERKEL, Daniel C., West Seneca New York 14224 (US)
(74) Representative: Crooks, Elizabeth Caroline
(86) International application number: PCT/US2010/043460
(87) International publication number: WO 2011/017138

(56) References cited:
- EP-A1- 0 644 173
- US-A- 4 158 737
- US-A- 4 404 124
- US-A- 4 762 956
- US-A- 4 800 075
- US-A- 4 940 687
- US-A- 6 022 823
- US-A- 6 022 823
- US-A1- 2002 038 065
- US-A1- 2006 217 579
- KNUNYANTS I L ET AL: "Reactions of fluoro olefins. XIII. Catalytic hydrogenation of perfluoro olefins", IZVESTIA AKADEMII NAUK SSSR. SERIA HIMICESKAA, RU, 1 January 1960 (1960-01-01), pages 1412 - 1418, XP009096304, ISSN: 0002-3353

## Description

### BACKGROUND

### 1. Field of Invention:

The present invention relates to hydrogenating fluoroolefins.

### 2. Description of Prior Art

Catalytic hydrogenation of fluoroolefins is frequently used in producing hydrofluorocarbons as useful products and/or intermediates. Various metals, such as Pd, supported on a substrate have long been recognized as highly effective hydrogenation catalysts. These catalysts are particularly effective in gas-phase reactions. Alumina is known as a support for these catalysts. Alumina has several different phases, typically designated by different Greek letters, e.g., alpha (α) (also known as corundum), beta (β), chi (χ), kappa (κ), eta (η), theta (θ), delta (δ), and gamma (λ). Each has a unique crystal structure and properties. For example, alpha alumina is composed of hexagonal crystals, whereas gamma alumina is composed of cubic crystals. (http://www.infoplease.com/ce6/sci/A0803541.html).

Aluminas other than alpha phase alumina are known as transitional phases because they can be transformed to the alpha form at high temperatures. *Id.* Other forms of alumina include amorphous alumina (that is, alumina lacking a crystalline structure) and activated alumina which is a highly porous form of dehydrated alumina that has a large specific surface area - often significantly over 200 square meters/g.(http://en.wikipedia.org/wiki/Activated alumina).

Typically, preferred supports are characterized by a high specific surface area. For example, US 2,657,980 states that "[i]n contrast with activated alumina is the well-known form of alumina known as corundum [alpha-alumina], which is not microporous and is unsuitable for [use as a hydrogenation catalyst]." *See also,* US 2,908,654 (Stating that, "It has not been feasible to employ corundum (sometimes called alpha alumina) as a carrier for highly reactive reforming catalyst, and in order to distinguish from corundum, catalyst carrier grades are designated by terms such as activated alumina, sorptive alumina or gamma alumina.") Amorphous alumina has also been reported as a support for hydrogenation catalysts (US 2,875,158).

Low concentration palladium/silver catalysts supported on alpha-alumina have been reported as hydrogenation catalyst for selectively hydrogenating acetylene (US 4,404,124) In contrast, other phases of alumina have been reported as hydrogenation catalyst for alkenes, particularly fluoroalkenes. For example, I. L Knunyants and E. I. Mysov (Kinetika i Kataliz, Vol. 8, No. 4, pp. 834-840) reported a Pd/Al₂O₃ catalyst with a specific surface of about 200 m²/g for the hydrogenation of CF₂=CF₂ to CHF₂CHF₂, and CF₃CF=CF₂ (HFP) to CF₃CHFCHF₂ (236ea). Based on the surface area information, the alumina used in this catalyst can be one of transition aluminas.

However, due to the occurrence of hydrogenolytic cleavage of the carbon-fluorine bond, a small amount of HF is generated during hydrogenation of fluoroolefin which can attack the transition alumina catalyst support causing catalyst structure change and catalyst deactivation. Thus, all known transition alumina supports for metal catalysts are inclined to be attacked by HF in the hydrogenation of fluoroolefins, thereby limiting the useful lifetime. Accordingly, there remains a need for a long-lived catalyst support for a catalyst useful in hydrogenating fluoroolefins. This invention satisfies this need among others.

US-4,762,956 discloses hydrogenating an impurity selected from acetylenic compounds, dienes and mixtures thereof. US-6,022,823 discloses producing supported palladium-gold catalysts. US-4,158,737 discloses an alumina-supported Group VIII noble metal catalyst and its use in the preparation of unsaturated organic esters. US-4,800,075 discloses a fixed-bed cyclic anthraquinone process for producing hydrogen peroxide wherein the hydrogenation catalyst comprises palladium or a mixture of palladium and platinum on a support containing alpha alumina. EP-0 644 173 discloses producing 1,1,1,2,3-pentafluoropropene from 1,1,1,2,3,3-hexafluoropropane by contacting gaseous hexafluoropropane with active carbon. US-2006/217579 discloses a catalyst for selectively hydrogenating acetylenic compounds to olefinic compounds, which comprises palladium supported upon an alumina support material and further comprises a lanthanide compound. Knunyants et al ("Reactions of fluoro olefins. XIII. Catalytic hydrogenation of perfluoro olefins", IZVESTIA AKADEMII NAUK SSSR. SERIA HIMICESKAA, RU, 1960-01-01, ISSN 0002-3353, PAGE 1412-1418) discloses the hydrogenation of various olefins under a variety of conditions.

### SUMMARY OF THE INVENTION

Applicants unexpectedly found that metal catalysts supported on alpha-alumina, which is the ultimate product of these transition aluminas under high temperature calcination and is characterized by small specific surface area (normally below 50 m²/g), provided stable activity for the hydrogenation of fluoroolefins, while those supported on transition alumina such as gamma-alumina exhibited unstable activity.

Accordingly, one aspect of this disclosure is a method, as defined in claim 1, for hydrogenating a fluoroolefi

The catalyst can be prepared by a method comprising (a) contacting at least one metal salt, at least one solvent, and alpha-alumina to form a slurry; (b) removing said solvent from said slurry to form a solvent-free powder; (c) optionally calcining said powder; (d) transforming said powder into a supported catalyst; and (e) contacting said support catalyst with a gaseous composition comprising H₂ to activate said supported catalyst, wherein said activated supported catalyst comprises 90 to 99.9 weight percent dehydrated alpha-alumina and 0.1 to 10 weight percent of a zero-valent metal derived from said metal salt, wherein the zero-valent metal is as defined above and in claim 1. In certain preferred embodiments, the catalyst can be produced by a method comprising the steps of (a) dissolving a salt of metal component in a suitable solvent to form a solution; (b) adding a suitable amount of alpha-alumina into said solution to form a slurry; (c) driving off the solvent from said slurry to form a paste; (d) drying said paste to form solvent-free powder; (e) calcining said solvent-free powder in N₂ flow for 2 to 8 hours at 300-500°C; (f) grinding the calcined powder to a finely divided state; (g) palletizing said fine powder into tablets; and (h) reducing said catalyst pellets in H₂ or diluted H₂ flow for 2 to 4 hours at 150-250°C prior to use. In an example of a method for preparing a catalyst comprising Pd, which is not a zero-valent metal of claim 1 and therefore is provided for illustrative purposes only, suitable salts of metal components are Pd(NO₃)₂ and PdCl₂.

### BRIEF DESCRIPTION OF THE DRAWING

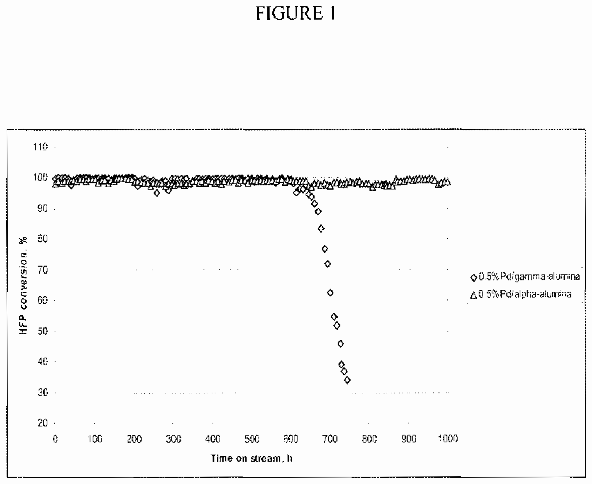

FIGURE 1 shows 1,1,1,2,3,3-hexafluoropropene (HFP) conversion versus time on stream during HFP hydrogenation over 0.5 wt% Pd/gamma-alumina and 0.5 wt% Pd/alpha-alumina catalysts (Pd is not a zero-valent metal of claim 1).

### DETAILED DESCRIPTION OF PREFERRED EMBODIMENTS OF THE INVENTION

According to the present disclosure, alpha-alumina supported metal catalysts are employed in the hydrogenation of fluoroolefins to hydro fluorocarbons, wherein the metal is Ru, Pt, Rh and Ir and any combination thereof. The metal loading can vary within a large range, from 0.1 - 10 wt %. However, the metal loading is preferably 0.1 to 5 wt %, and more preferably 0.1 to 1 wt %. It has been discovered that supported catalyst having metal concentrations below 0.1 wt. % are not highly effective at hydrogenating fluoroolefins or hydrofluoroolefins.

The catalyst can be prepared by a method in which a salt of a metal component is added to an amount of solvent sufficient to substantially dissolve or solubilize the metal salt. In an example of a method for preparing a catalyst comprising Pd, which is not a zero-valent metal of claim 1 and therefore is provided for illustrative purposes only, suitable salts of metal components are Pd(NO₃)₂ and PdCl₂. The preferred solvent is one in which the metal salt is readily soluble. The choice of solvent may vary depending on the particular metal salts. Examples of solvents that can be used for the preparation of the catalyst compositions of the present invention include water, alcohols, ethers, and mixtures thereof. Useful alcohols include monohydric and polyhydric alcohols. Most preferred alcohols are those that are monohydric and have 1 to 5 carbon atoms. A most preferred solvent is water.

Desired amount of alpha-alumina powder is added to the solution of said metal salt to form a slurry. After formation of the slurry, substantially all of the solvent is removed to form a solid mass of a mixture of said metal salt and said alpha-alumina. Although the solvent can be removed in one step, a preferred method is to drive off a portion of the solvent from the slurry to form a paste and then followed by drying the paste to form the solid mass. Any conventional technique can be used to drive off the solvent. Examples of such techniques include vigorous stirring at room or elevated temperatures, evaporation, settling and decanting, centrifugation, and filtration. It is preferred to evaporate off a desired amount of solvent to form the paste. The paste is then dried by any suitable method to form a free-flowing, substantially solvent-free powder. Preferred methods for drying include oven drying, most preferably at temperatures from 110 °C to 120 °C, and spray drying. As used herein, the term "solvent free" means that less than 1 wt. %, preferably 0.5 wt % or less, more preferably 0.1 wt % or less, and most preferably no solvent will remain with the powder after solvent removal/drying. Upon removal of solvent, the powder will take the form of a solid mass (or powder) of a mixture of particles of said metal salt and said alpha-alumina.

Optionally, the solid mass of the mixture of said metal salt and said alpha-alumina powder is then calcined. Calcination is preferably carried out at a temperature of 100 °C to 750 °C, more preferably at a temperature of 200 °C to 600 °C, and most preferably at a temperature of 300 °C to 500 °C. Calcination may further optionally be carried out in the presence of an inert gas, such as nitrogen or argon.

After calcination, the powder is optionally further grinded such that it becomes more finely-divided. The powder is further optionally pelletized in order to form pellets.

The catalyst pellets are then loaded into a reactor and prior to use are reduced in hydrogen or diluted hydrogen flow for 2 - 4 hours at a temperature of 50 to 500 °C, more preferably at a temperature of 100 to 300 °C, and most preferably at a temperature of 150 to 250 °C.

Although it is contemplated that the hydrogenation of fluoroolefins may be conducted in batch operation, it is preferred that the hydrogenation reaction is carried out as a substantially continuous operation. Furthermore, while it is possible that the hydrogenation reaction may involve in certain embodiments a liquid phase reaction, it is contemplated that in preferred embodiments the hydrogenation reaction comprises, and even more preferably consists of, at least two vapor phase reaction stages.

With respect to the number of reaction stages, applicants have found surprisingly and unexpectedly that overall reaction conversion and selectivity can be achieved at relatively high levels by the use of at least two reaction stages wherein the first stage of reaction is conducted under conditions effective to achieve a first, relatively low rate of conversion to produce a first stage reaction effluent, and at least a second stage of reaction which is fed by at least a portion of said first stage effluent and which is conducted under conditions effective to achieve a second rate of conversion higher than said first rate. Preferably, reaction conditions are controlled in each of the first and second stages in order to achieve the desired conversion in accordance with the present invention. As used herein, the term "reaction conditions" is intended to include the singular and means control of any one or more processing parameters which can be modified by the operator of the reaction to produce the conversion of the feed material in accordance with the teachings contained herein. By way of example, but not by way of limitation, conversion of the feed material may be controlled or regulated by controlling or regulating any one or more of the following: the temperature of the reaction, the flow rate of the reactants, the presence of diluent, the amount of catalyst present in the reaction vessel, the shape and size of the reaction vessel, the pressure of the reaction, and any combinations of these and other process parameters which will be available and known to those skilled in the art in view of the disclosure contained herein.

Applicants have found that in preferred embodiments the step of controlling the conversion in the first stage of the hydrogenation reaction is achieved by judicious selection and control of the amount of catalyst present in the first stage of reaction relative to the feed rate of one or more of the reactants and/or by judicious selection and control of the reaction temperature, and preferably by judicious selection and control of both of these process parameters. The step of judiciously selecting the amount of catalyst to be used in the first stage of reaction includes the step of estimating the amount of catalyst theoretically needed to convert 100% of the feed material. Such an estimate can be obtained by any and all known methods for making such an estimate, which should be apparent to those skilled in the art in view of the teachings contained herein. In addition, the step of judiciously selecting the amount of catalyst may also involve conducting bench, pilot or similar studies to determine the amount of the particular catalyst being used which is needed to convert 100% of the feed material under the feed rate in other process parameters which have otherwise been chosen. Based upon this estimate, the preferred embodiments of the present invention then include the step of providing in the first stage of reaction an amount of catalyst that is substantially below the amount required for 100% conversion, and even more preferably is sufficiently low so as to result in a conversion of the feed olefin of from 10% to 60 %, more preferably from 10% to 40 %, and even more preferably from 10% to 25%. Once again, those skilled in the art will appreciate that the step of judiciously choosing the amount of catalyst may further include running additional bench, pilot or other studies with the reduced amount of catalyst and adjusting the amount of catalyst accordingly. It is contemplated that all such studies and estimates can be achieved without undue experimentation in view of the teachings contained herein.

Applicants have found that the step of maintaining a relatively low conversion of reactant in accordance with the present invention in a first stage of reaction has an advantageous affect on the selectivity of the reaction to the desired hydrofluorocarbon. In other words, although the amount of conversion which occurs in the first stage of reaction is controlled to be well below that which is desired for the overall hydrogenation step, applicants have found that an improved, higher percentage of the feed material is converted to the desired hydrofluorocarbon in the first reaction stage (that is, improved selectivity is achieved) by controlling the conversion as described herein. More specifically, it is preferred in many embodiments that the selectivity to the desired hydrofluorocarbon in the first reaction stage is at least 80%, more preferably at least 90 %, and even more preferably at least 95%, and in many preferred embodiments 97% or greater.

In certain preferred embodiments the step of controlling the conversion in the first reaction stage further includes removing heat from the reaction by cooling at least a portion of the reaction mixture. It is contemplated that those skilled in the art will be able to devise without undue experimentation and many means and mechanisms for attaining such cooling in view of the teachings contained herein and all such means and mechanisms are with the scope of the present invention.

In preferred embodiments, at least a portion of the effluent from the first reaction stage is fed directly, or optionally after some further processing, to a second reaction stage in which the unreacted fluoroolefin remaining in the effluent after the first reaction stage is converted to the hydrofluorocarbon in accordance with the present invention. More specifically is preferred that the second reaction stage or subsequent reaction stages if present, is operated under conditions effective to convert the fluoroolefin contained in the feed stream to the second reactor stage at a conversion rate that is greater than, and preferably substantially greater than, the conversion percentage in the first reaction stage. In certain preferred embodiments, for example, the conversion percentage in the second reaction stage is from 20% to 99%, depending in large part upon the total number of reactant stages used to affect the overall conversion step. For example, in embodiments consisting of a two-stage reaction system, it is contemplated that the conversion in the second reaction stage is preferably greater than 95%, and even more preferably 100%. However, as those skilled in the art will appreciate from the teachings contained herein, such a two-stage reaction may not be sufficient to produce the desired selectivity to the hydro fluorocarbon. In such cases, it is within the scope of the present invention that the conversion step may comprise greater than two reaction stages, including in some embodiments as many 10 or more reaction stages.

The size and shape, and other characteristics of the reaction vessel itself may vary widely with the scope of the present invention, and it is contemplated that the vessel associated with each stage may be different than or the same as the vessel associated with the upstream and downstream reaction stages. Furthermore, it is contemplated that all reaction stages can occur inside a single vessel, provided that means and mechanisms necessary to control conversion are provided. For example, it may be desirable in certain embodiments to utilize a single tubular reactor for each reaction stage, providing conversion control by judicious selection of the amount and/or distribution of catalyst throughout the tubular reactor. In such a case, it is possible to further control the conversion in different sections of the same tubular reactor by controlling the amount of heat removed from or added to different sections of the tubular reactor.

The catalyst compositions disclosed in the present invention are useful in converting fluoroolefins to hydrofluorocarbons. The catalysts are stable because of their resistance to HF attack and can be re-used after regeneration. One or more of the hydrogenation catalyst disclosed in the present invention may be used for one or more of the reaction stages in accordance with the present invention.

Thus, certain embodiments of the present methods comprise bringing a fluoroolefin and a hydrogenation agent, H₂, into contact with a first amount of catalyst in a first reaction stage to produce a reaction stream comprising hydrofluorocarbon(s), unreacted fluoroolefin and hydrogen; contacting at least a portion of this first effluent stream with a second amount of catalyst in a second stage of reaction to produce a hydrofluorocarbon, wherein the second amount of catalyst is greater than the first amount of catalyst and wherein conversion to the fluoroolefin is higher in the second stage of reaction.

Table 1 sets forth examples of hydro fluorocarbons and fluoroolefins from which they can be obtained (fluoroolefin in left column and corresponding hydrofluorocarbon in the right column).

**Table 1**

| **Fluoroolefins** | **Hydrofluorocarbons** |
|---|---|
| 1,1,2,3,3,3-hexafluoropropene CF₃CF=CF₂ (1216) | 1,1,1,2,3,3-hexafluoropropane CF₃CHFCHF₂ (236ea) |
| 1,2,3,3,3-pentafluoropropene CF₃CF=CHF (Z/E-1225ye) | 1,1,1,2,3-pentafluoropropane CF₃CHFCH₂F (245eb) |
| 1,1,3,3,3-pentafluoropropene CF₃CH=CF₂ (1225zc) | 1,1,1,3,3-pentafluoropropane CF₃CH₂CHF₂ (245fa) |
| 1,3,3,3-tetrafluoropropene CF₃CH=CHF (trans/cis-1234ze) | 1,1,1,3-tetrafluoropropane CF₃CH₂CH₂F (254fb) |
| 2,3,3,3-tetrafluoropropene CF₃CF=CH₂ (1234yf) | 1,1,1,2-tetrafluoropropane CF₃CHFCH₃ (254eb) |

### EXAMPLES

The following reference examples illustrate the effects provided by the claimed invention and are not to be construed as limiting.

### Reference Example 1: Comparison of gamma-alumina and alpha-alumina supported Pd catalysts for 1,1,1,2,3,3-hexafluoropropene hydrogenation

0.5 wt% Pd/gamma-alumina and 0.5 wt% Pd/alpha-alumina, which have a specific surface area of 243 and 33 m²/g, respectively, were compared for 1,1,1,2,3,3-hexafluoropropene (HFP) hydrogenation. About 2 g of catalyst diluted with 20 ml of Monel packing was charged into a ¾" Monel tube reactor and was in-situ reduced in 10% H₂/N₂ flow for 2 hours at 200°C. HFP was fed into reactor at a rate of 5 g/h, and H₂ was co-fed according to a mole ratio of H₂/HFP equal to 1.5. As shown in Table 2, both catalysts initially provided a near complete HFP conversion and a 236ea selectivity of above 99.5%. Nevertheless, as shown in Figure 1, while no deactivation was noted over the 0.5 wt% Pd/alpha-alumina catalyst even after 1000 h on stream, rapid deactivation was observed over the 0.5 wt% Pd/gamma-alumina beginning around 600 h on stream. This indicates that the alpha-alumina supported Pd catalyst is much more stable than the gamma-alumina supported Pd catalyst.

**Table 2 HFP hydrogenation over alumina supported Pd catalysts***

| Catalyst | Temp. (°C) | Conversion, % HFP | Selectivity, % 236ea | Selectivity, % others |
|---|---|---|---|---|
| 0.5% Pd/γ-alumina | 100 | 99.9 | 99.6 | 0.4 |
| 0.5% Pd/α-alumina | 100 | 98.2 | 99.9 | 0.1 |
| * data obtained after 2 h on stream. | | | | |

### Reference Example 2: Hydrogenation of 1,1,1,2,3,3-hexafluoropropene over alpha-alumina supported Pd catalyst

0.5 wt% Pd/alpha-alumina catalyst, which has a specific surface area of 33 m²/g, was used for 1,1,1,2,3,3-hexafluoropropene (HFP) hydrogenation. About 1 g of catalyst diluted with 10 ml of Monel packing was charged into a ¾" Monel tube reactor and was in-situ reduced in 10% H₂/N₂ flow for 2 hours at 200°C. HFP was fed into reactor at a rate of 65 g/h, and H₂ was co-fed according to a mole ratio of H₂/HFP equal to 1.5. GC analysis of the product stream showed that the catalyst provided an HFP conversion of around 55% and a 245eb selectivity of about 99.5%. No deactivation was noted during the period of time of the test which lasted for 800 hours, indicating the alpha-alumina supported Pd catalyst can provide stable activity for HFP hydrogenation.

0.5 wt% Pd/alpha-alumina catalyst, which has a specific surface area of 33 m²/g, was used for 1,1,1,2,3-pentafluoropropene (1225ye) hydrogenation. About 0.5 g of catalyst diluted with 10 ml of Monel packing was charged into a ¾" Monel tube reactor and was in-situ reduced in 10% H₂/N₂ flow for 2 hours at 200°C. 1225ye was fed into reactor at a rate of 30 g/h, and H₂ was co-fed according to a mole ratio of H₂/1225ye equal to 1.5. GC analysis of the product stream showed that the catalyst provided a 1225ye conversion of around 45% and a 245eb selectivity of about 98.5%. No deactivation was noted during the period of time of the test which lasted for 800 hours, indicating the alpha-alumina supported Pd catalyst can provide stable activity for 1225ye hydrogenation.

## Claims

1. A method for hydrogenating a compound comprising:
contacting a reactant comprising a fluoroolefin, with a supported hydrogenation catalyst under reaction conditions effective to form a reaction product comprising a hydrogenated derivative of said olefin; the method comprising the steps of:
a) adding hydrogen and a fluoroolefin to a reaction vessel containing the hydrogenation catalyst; and
b) reacting said fluoroolefin with hydrogen over said hydrogenation catalyst to produce a hydrofluorocarbon;
wherein said fluoroolefin reactant and said hydrofluorocarbon reaction product are selected from the following combinations:
1,1,2,3,3,3-hexafluoropropene (FO-1216; HFP) and 1,1,1,2,3,3-hexafluoropropane (HFC-236ea);
1,2,3,3,3-pentafluoropropene (HFO-1225ye) and 1,1,1,2,3-pentafluoropropane (HFC-245eb);
1,1,3,3,3-pentafluoropropene (HFO-1225zc) and 1,1,1,3,3-pentafluoropropane (HFC-245fa);
1,3,3,3-tetrafluoropropene (HFO-1234ze) and 1,1,1,3-tetrafluoropropane (HFC-254fb); and
2,3,3,3-tetrafluropropene (HFO-1234yf) and 1,1,1,2-tetrafluoropropane (HFC-254eb);
and
wherein said supported hydrogenation catalyst comprises:
a) 90 to 99.9 weight % of alumina, wherein said alumina is at least 90 weight % alpha-alumina; and
b) 0.1 to 10 weight % of at least one zero-valent metal, wherein said at least one zero-valent metal is selected from the group consisting of Ru, Pt, Rh and Ir.

2. A method according to claim 1, wherein the metal loading is 0.1 to 5 weight %.

3. A method according to claim 1 or claim 2, wherein the metal loading is 0.1 to 1 weight %.

4. A method according to claim 1, wherein said hydrogenation reaction comprises at least two vapour phase reaction stages.

## Patentansprüche

1. Verfahren zur Hydrierung einer Verbindung, das Folgendes umfasst:
das Inkontaktbringen eines Reaktanten, der ein Fluorolefin umfasst, mit einem geträgerten Hydrierkatalysator unter Reaktionsbedingungen, unter denen ein Reaktionsprodukt, das ein hydriertes Derivat des Olefins umfasst, gebildet wird; wobei das Verfahren die folgenden Schritte umfasst:
a) Zugeben von Wasserstoff und einem Fluorolefin zu einem Reaktionsgefäß, das den Hydrierkatalysator enthält; und
b) Umsetzen des Fluorolefins mit Wasserstoff an dem Hydrierkatalysator zur Bildung eines Fluorkohlenwasserstoffs;
wobei der Fluorolefin-Reaktant und der als Reaktionsprodukt anfallende Fluorkohlenwasserstoff aus den folgenden Kombinationen ausgewählt sind:
1,1,2,3,3,3-Hexafluorpropen (FO-1216; HFP) und 1,1,1,2,3,3-Hexafluorpropan (H-FKW 236ea);
1,2,3,3,3-Pentafluorpropen (HFO-1225ye) und 1,1,1,2,3-Pentafluorpropan (H-FKW 245eb);
1,1,3,3,3-Pentafluorpropen (HFO-1225zc) und 1,1,1,3,3-Pentafluorpropan (H-FKW 245fa);
1,3,3,3-Tetrafluorpropen (HFO-1234ze) und 1,1,1,3-Tetrafluorpropan (H-FKW 254fb); und
2,3,3,3-Tetrafluropropen (HFO-1234yf) und 1,1,1,2-Tetrafluorpropan (H-FKW 254eb);
und
wobei der geträgerte Hydrierkatalysator Folgendes umfasst:
a) 90 bis 99,9 Gew.-% Aluminiumoxid, wobei es sich bei dem Aluminiumoxid um mindestens 90 Gew.-% alpha-Aluminiumoxid handelt; und
b) 0,1 bis 10 Gew.-% mindestens eines nullwertigen Metalls, wobei das mindestens eine nullwertige Metall aus der Gruppe bestehend aus Ru, Pt, Rh und Ir ausgewählt ist.

2. Verfahren nach Anspruch 1, bei dem die Metallbeladung 0,1 bis 5 Gew.-% beträgt.

3. Verfahren nach Anspruch 1 oder Anspruch 2, bei dem die Metallbeladung 0,1 bis 1 Gew.-% beträgt.

4. Verfahren nach Anspruch 1, bei dem die Hydrierungsreaktion mindestens zwei Dampfphasen-Reaktionsstufen umfasst.

## Revendications

1. Procédé d'hydrogénation d'un composé comprenant :
la mise en contact d'un réactif comprenant une fluoro-oléfine, avec un catalyseur d'hydrogénation sur support dans des conditions de réaction efficaces pour former un produit de réaction comprenant un dérivé hydrogéné de ladite oléfine ; le procédé comprenant les étapes de :
a) ajout d'hydrogène et d'une fluoro-oléfine dans une cuve de réaction contenant le catalyseur d'hydrogénation ; et
b) réaction de ladite fluoro-oléfine avec de l'hydrogène sur ledit catalyseur d'hydrogénation pour produire un hydrofluorocarbone ;
dans lequel ledit réactif fluoro-oléfine et ledit produit de réaction hydrofluorocarbone sont choisis parmi les combinaisons suivantes :
1,1,2,3,3,3-hexafluoropropène (FO-1216 ; HFP) et 1,1,1,2,3,3-hexafluoropropane (HFC-236ea) ;
1,2,3,3,3-pentafluoropropène (HFO-1225ye) et 1,1,1,2,3-pentafluoropropane (HFC-245eb) ;
1,1,3,3,3-pentafluoropropène (HFO-1225zc) et 1,1,1,3,3-pentafluoropropane (HFC-245fa) ;
1,3,3,3-tétrafluoropropène (HFO-1234ze) et 1,1,1,3-tétrafluoropropane (HFC-254fb) ; et
2,3,3,3-tétrafluropropène (HFO-1234yf) et 1,1,1,2-tétrafluoropropane (HFC-254eb) ;
et
dans lequel ledit catalyseur d'hydrogénation sur support comprend :
a) 90 à 99,9 % en poids d'alumine, ladite alumine étant au moins 90 % en poids d'alpha-alumine ; et
b) 0,1 à 10 % en poids d'au moins un métal de valence zéro, ledit au moins un métal de valence zéro étant choisi dans le groupe constitué de Ru, Pt, Rh et Ir.

2. Procédé selon la revendication 1, dans lequel la charge de métal est de 0,1 à 5 % en poids.

3. Procédé selon la revendication 1 ou la revendication 2, dans lequel la charge de métal est de 0,1 à 1 % en poids.

4. Procédé selon la revendication 1, dans lequel ladite réaction d'hydrogénation comprend au moins deux étapes de réaction en phase vapeur.
